# EUROPÄISCHE PATENTSCHRIFT

(11) **EP 2 750 720 B1**
(45) Veröffentlichungstag und Bekanntmachung des Hinweises auf die Patenterteilung: **30.09.2015**
(21) Anmeldenummer: 12745862.8
(22) Anmeldetag: 10.08.2012
(51) Int. Cl.: A61L 9/01, C11D 3/50, A61L 9/14, C07D 498/08

(54) **VERFAHREN ZUR FEHLGERUCHSBEKÄMPFUNG UNTER VERWENDUNG VON OXAZOLIDINEN**
PROCESS FOR CONTROLLING MALODORS USING OXAZOLIDINES
PROCÉDÉ DE LUTTE CONTRE LES MAUVAISES ODEURS EN UTILISANT DES OXAZOLIDINES

(30) Priorität: 31.08.2011 DE 102011081871
(43) Veröffentlichungstag der Anmeldung: 09.07.2014
(73) Patentinhaber: Henkel AG&Co. KGAA, 40589 Düsseldorf (DE)
(72) Erfinder: HUCHEL, Ursula, 50670 Köln (DE); WEYHE, Marc, 47802 Krefeld (DE); BARON, Lukas, 45307 Essen (DE)
(86) Internationale Anmeldenummer: PCT/EP2012/065638
(87) Internationale Veröffentlichungsnummer: WO 2013/029963

(56) Entgegenhaltungen:
- EP-A1- 1 787 689
- WO-A1-2007/087977
- WO-A1-2010/142479
- DE-A1-102010 002 106

## Beschreibung

Die vorliegende Erfindung betrifft ein Verfahren zum Abbau von Fehlgerüchen, vorzugsweise mit Blick auf die Behandlung harter und/oder weicher Oberflächen, insbesondere betrifft es den Abbau von Fehlgerüchen im Rahmen eines Textilbehandlungsverfahrens und ebenso den Abbau von Fehlgerüchen in der Raumluft.

Ein wichtiges Verbraucherbedürfnis, welches z.B. auch bei der Anwendung von Wasch-, Reinigungs- oder Pflegemitteln eine Rolle spielt, besteht in der Beseitigung oder zumindest in der Verminderung von Schlechtgerüchen (d.h. Fehlgerüchen) oder unerwünschten Gerüchen. Fehlgerüche gehen von bestimmten geruchsaktiven Verbindungen aus, welche auch als Stinkstoffe bezeichnet werden. Stinkstoffe sind übel riechende Verbindungen mit sogenannten kakosmophoren Gruppen, z.B. Amin-Derivate und Schwefel-Derivate. Die Anwesenheit solcher Fehlgerüche führt in der Regel zu einer Beeinträchtigung des menschlichen Wohlbefindens, weshalb der Verbraucher die Auslöschung dieser Gerüche anstrebt. Allerdings werden die Fehlgerüche oftmals nicht ausgelöscht, sondern lediglich überdeckt. Dazu werden gewöhnlich Produkte eingesetzt, welche flüchtige, meist angenehm riechende Stoffe enthalten und welche bereits in kleinen Mengen üble Gerüche überdecken können.

In US 6,861,402 sind Riechstoffvorläufer beschrieben, die einen Riechstoff-Aldehyd oder ein Riechstoff-Keton in Form eines Oxazolidins gebunden enthalten. Dabei wird beispielsweise N-Benzolethanolamin mit einem Riechstoff umgesetzt, so dass sich ein monocyclisches Oxazolidin ergibt. In US 2003/0207786 A1 sind ebenfalls Riechstoffvorläufer beschrieben, die eine Oxazolidinstruktur aufweisen. In US 4,277,353 werden mono- und bicyclische Oxazolidine als korrosionsinhibierende Additive für Schmieröle beschrieben. In US 2004/0087453 A1 werden bestimmte photolabile Riechstoffvorläufer beschrieben, welche auch in Form von Oxazolidinen gebunden sein können. In US 2004/0067870 A1 werden spezielle Riechstoff-Aldehyde mit einem tertiären alpha-Kohlenstoffatom beschrieben, welche auch in Form von Oxazolidinen gebunden sein können. In US 2003/0158079 A1 werden Wirkstoffabgabesysteme, geeignet zum Abgeben eines Wirkstoffs an ein Substrat, beschrieben, wobei das Wirkstoffabgabesystem einen Wirkstoff in der Form eines Aldehyds oder Ketons und ein Amin umfasst, das eine primäre und/oder sekundäre Amineinheit umfasst. In WO2007/087977 A1 werden 1-Aza-3,7-dioxabicyclo[3.3.0]octan-Verbindungen, deren Verwendung als Riechstoffvorläufer und deren Einsatz in Wasch- oder Reinigungsmitteln beschrieben. Ein Verfahren zum Abbau von Fehlgerüchen wird dort allerdings nicht erwähnt.

In WO2010/094356 A1 werden bestimmte Copolymere beschrieben, wobei eines der eingesetzten Monomere eine 1-Aza-3,7-dioxabicyclo[3.3.0]octan-Verbindung ist. Diese Copolymere werden als Riechstoffvorläufer eingesetzt. Ein Verfahren zum Abbau von Fehlgerüchen wird dort allerdings nicht erwähnt.

In WO2008/074598 A1 werden Kieselsäureester beschrieben, an welche Duftstoffe als 1-Aza-3,7-dioxabicyclo[3.3.0]octan-Verbindungen gebunden sind. Ein Verfahren zum Abbau von Fehlgerüchen wird dort allerdings nicht erwähnt.

Aufgabe der vorliegenden Erfindung war es, dem Verbraucher eine weitere Möglichkeit zu geben, einen Abbau von Fehlgerüchen herbeizuführen.

Gegenstand der Erfindung ist ein Verfahren zum Abbau von Fehlgerüchen durch Einsatz einer 1-Aza-3,7-dioxabicyclo[3.3.0]octan-Verbindung der allgemeinen Formel (I) 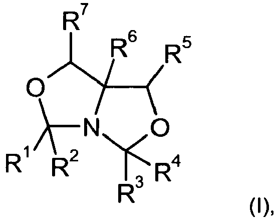 wobei
- R¹, R², R³, R⁴: unabhängig voneinander für Reste stehen, die in einer Verbindung der allgemeinen Formel R¹-C(=O)-R² oder R³-C(=O)-R⁴ einen Riechstoff-Aldehyd mit mindestens 6, vorzugsweise mindestens 7, insbesondere mindestens 8 Kohlenstoffatomen oder ein Riechstoff-Keton mit mindestens 6, vorzugsweise mindestens 7, insbesondere mindestens 8 Kohlenstoffatomen ergeben,
- R⁵, R⁶, R⁷: unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann.

Alle Kohlenwasserstoff-Reste im Sinne der Erfindung können grundsätzlich acyclisch oder cyclisch, substituiert oder unsubstituiert, verzweigt oder unverzweigt sowie gesättigt oder ungesättigt sein. Die Kohlenwasserstoff-Reste im Sinne der Erfindung können grundsätzlich Heteroatome, wie z.B. Stickstoff-, Sauerstoff- oder Schwefel-Atome umfassen. Bevorzugt mit Blick auf R⁵, R⁶ und R⁷ sind jeweils acyclische, unverzweigte Kohlenwasserstoffreste, die ggf. substituiert sein können. Geeignete Substituenten sind z.B. Hydroxy-, Alkoxy-, Amino- oder Halogen-Gruppen.

Der Begriff "Einsatz" ist hier im weitesten Sinne zu verstehen, d.h. es wird eine 1-Aza-3,7-dioxabicyclo[3.3.0]octan-Verbindung der allgemeinen Formel (I) eingesetzt, um die genannten Fehlgerüche abzubauen. Fehlgerüche abzubauen, geht über das bloße Überdecken von Fehlgerüchen hinaus und meint die Deaktivierung der Fehlgeruch auslösenden Stoffe. Deaktivierung bedeutet hier, dass der Fehlgeruch zumindest vermindert und insbesondere sogar gänzlich beseitigt, also ausgelöscht wird. Immer wenn ein Fehlgeruch vorliegt, muss es ein Objekt geben, von dem dieser Fehlgeruch ausgeht oder einen Raum oder ein System, in welchem der Fehlgeruch wahrnehmbar ist. Zu diesem Objekt oder in diesen Raum oder in das System kann erfindungsgemäß eine 1-Aza-3,7-dioxabicyclo[3.3.0]octan-Verbindung der allgemeinen Formel (I) zugegeben werden, um den unerwünschten Geruch abzubauen. Dabei kann die Zugabe der 1-Aza-3,7-dioxabicyclo[3.3.0]octan-Verbindung der allgemeinen Formel (I) z.B. durch Sprühen oder im Rahmen eines Wasch- oder Reinigungsprozesses erfolgen.

Die 1-Aza-3,7-dioxabicyclo[3.3.0]octan-Verbindung der allgemeinen Formel (I) kann z.B. in die Raumluft gesprüht werden, sie kann z.B. in einen Behälter mit Wasser oder einer Waschlauge gegeben werden, sie kann direkt auf jedes abgegrenzte Objekt, also z.B. auch ein verschwitztes Hemd oder eine Vielzahl solcher Objekte, wie z.B. ein Waschgut bestehend aus vielen verschmutzten Hemden oder ähnlichem, gegeben werden. Es konnte überraschend gefunden werden, dass das erfindungsgemäße Verfahren einen deutlichen Abbau bis hin zur Auslöschung von Fehlgerüchen ermöglicht.

Das erfindungsgemäße Verfahren ist insbesondere zur Minimierung oder Auslöschung von Fehlgerüchen mit Blick auf übelriechende Textilien, die z.B. nach sportlicher Aktivität resultieren oder mit Blick auf Fehlgerüche im Toiletten- oder WC-Bereich erfolgreich einsetzbar.

Erfinderseitig wird vermutet, dass das Funktionsprinzip der eingesetzten 1-Aza-3,7-dioxabicyclo[3.3.0]octan-Verbindung bei der Fehlgeruchsbeseitigung auch darauf beruht, dass diese genannte Verbindung durch Hydrolyse neben Riechstoff-Aldehyden und/oder-ketonen auch Aminoalkohole freisetzt, wodurch eine Deaktivierung von Stinkstoffen ermöglicht wird. Überraschenderweise konnte aber gefunden werden, dass die eingesetzten 1-Aza-3,7-dioxabi-cyclo[3.3.0]octanVerbindungen der allgemeinen Formel (I) eine bessere Fehlgeruchsbeseitigung ermöglichen als die ansonsten vergleichbare Kombination separat eingesetzter Aminoalkohole und Riechstoffe, d.h. Riechstoff-Aldehyde und/oder -ketone, also die Zerfallsprodukte der 1-Aza-3,7-dioxabicyclo[3.3.0]octan-Verbindung. Eine bessere Fehlgeruchsbeseitigung gegenüber einzeln eingesetztem Aminoalkohol oder Riechstoff wird ohnehin erreicht. Die überlegene Wirkung konnte insbesondere auch für Anwendungen in der Waschmaschine gefunden werden. Besonders vorteilhaft ist die Freisetzung von Riechstoff-Aldehyden oder -ketonen und Aminoalkoholen durch Hydrolyse beispielsweise beim Schwitzen, wodurch der sich bildende Schlechtgeruch unmittelbar bei seiner Entstehung abgefangen werden kann.

Als besonders vorteilhaft bei der Fehlgeruchsbekämpfung im Sinne der Erfindung hat sich erwiesen, das erfindungsgemäße Verfahren bei Anwesenheit katalytischer Mengen an Säuren, vorzugsweise Lewis- und/oder Brönsted-Säuren, insbesondere Brönsted-Säuren durchzuführen.

Bereits katalytische Mengen an Wasser, z.B. resultierend aus der üblichen Luftfeuchtigkeit oder Umgebungsluft, reichen aus, um einen besonders effizienten Abbau von Fehlgerüchen im Sinne der Erfindung zu gewährleisten.

Der Einfluss der Umgebungsluft ist im Sinne der Erfindung auch deshalb günstig, weil diese in der Regel leicht sauer ist. Das liegt daran, dass in der Feuchtigkeit der Luft, also dem in Luft vorhandenen Wasser, Kohlendioxid zum Teil gelöst wird, welches immer in der Luft vorhanden ist, und sich so formal Kohlensäure bildet, so dass die H⁺-Ionenkonzentration dieser Feuchtigkeit steigt. In der Umgebungsluft und der darin enthaltenen Luftfeuchte sind also in der Regel katalytische Mengen an Säuren enthalten.

Wie bereits klargestellt wurde, stehen in Formel (I) die Reste R¹, R², R³, R⁴ unabhängig voneinander für Reste, die in einer Verbindung der allgemeinen Formel R¹-C(=O)-R² oder R³-C(=O)-R⁴ einen Riechstoff-Aldehyd mit mindestens 6 Kohlenstoffatomen oder ein Riechstoff-Keton mit mindestens 6 Kohlenstoffatomen ergeben.

Vorzugsweise liegen maximal in einem der Strukturelemente -CR¹R² oder -CR³R⁴ Reste R¹ und R² oder R³ und R⁴ vor, die in einer Verbindung der allgemeinen Formel R¹-C(=O)-R² oder R³-C(=O)-R⁴ ein Riechstoff-Keton ergeben. Insbesondere liegen in beiden Strukturelementen -CR¹R² und -CR³R⁴ Reste R¹ und R² sowie R³ und R⁴ vor, die in einer Verbindung der allgemeinen Formel R¹-C(=O)-R² sowie R³-C(=O)-R⁴ jeweils ein Riechstoff-Aldehyd ergeben, insbesondere das gleiche Riechstoff-Aldehyd.

Der Riechstoff-Aldehyd ist gemäß einer bevorzugten Ausführungsform der Erfindung ausgewählt aus Adoxal (2,6,10-Trimethyl-9-undecenal), Anisaldehyd (4-Methoxybenzaldehyd), Cymal (3-(4-Isopropyl-phenyl)-2-methylpropanal), Ethylvanillin, Florhydral (3-(3-isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Di-methyl-3-cyclohexen-1-carboxaldehyd (Triplal), 4-Methoxy-benzaldehyd, Benzaldehyd, 3-(4-tert-Butylphenyl)-propanal, 2-Methyl-3-(para-methoxyphenyl)-propanal, 2-Methyl-4-(2,6,6-timethyl-2(1)-cyclo-hexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)-oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-car-boxaldehyd, 2-Methyl-3-(isopropyl-phenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pen-tenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarbox-aldehyd, 7-Hy-droxy-3,7-dimethyl-octanal, trans-4-Decenal, 2,6-Nonadienal, para-Tolyl-acetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacet-aldehyd, 5,9-Di-methyl-4,8-decadienal, Päonienaldehyd (6,10-Dimethyl-3-oxa-5,9-undecadien-1-al), Hexahydro- 4,7-methanindan-1-carboxaldehyd, 2-Methyloctanal, alpha-Methyl-4-(1-methylethyl)benzolace-taldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

Das Riechstoff-Keton ist gemäß einer bevorzugten Ausführungsform der Erfindung ausgewählt aus Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-Ionon, betalonon, gamma-Methyl-Ionon, Fleuramon (2-heptylcyclopen-tanon), Dihydrojasmon, cis-Jasmon, iso-E-Super (1-(1,2,3,4,5,6,7,8-octahydro-2,3,8,8-tetramethyl-2-naphthalenyl)-ethan-1-on (und I-somere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldecahydro-2-naphton, Dimethyloctenon, Frescomenthe (2-butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-benzodioxol-5-yl) bu-tan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amyl-cyclohexanon), 4-tert-butyl cyclohexanon, Delphon (2-pentyl-cyclopentanon), Muscon (CAS 541-91-3), Neobutenon (1-(5,5-dimethyl-1-cyclohexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on sowie Tetrameran (6,10-dimethylundecen-2-on).

R⁵ und R⁷ stehen gemäß einer bevorzugten Ausführungsform der Erfindung, unabhängig voneinander, vorteilhafterweise jeweils für Wasserstoff oder einen C₁₋₆-Kohlenwasserstoffrest, der ggf. substituiert sein kann, vorzugsweise C₁₋₃- Kohlenwasserstoffrest. Besonders bevorzugt sind R⁵ und R⁷ jeweils Wasserstoff oder jeweils ein Methyl- oder Ethylrest, insbesondere aber jeweils Wasserstoff.

R¹ und R³ stehen gemäß einer bevorzugten Ausführungsform der Erfindung, unabhängig voneinander, vorteilhafterweise jeweils für einen C₆₋₂₄-Kohlenwasserstoffrest, vorzugsweise C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein kann.

In einer weiter bevorzugten Ausführungsform bedeuten R², R⁴, R⁵, R⁷ Wasserstoff, R¹ und R³ bedeuten jeweils einen C₆₋₂₄-Kohlenwasserstoffrest, vorzugsweise C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein kann, und R⁶ steht für Wasserstoff oder einen C₁₋₂₄-Kohlenwasserstoffrest, welcher acyclisch oder cyclisch, substituiert oder unsubstituiert, verzweigt oder unverzweigt sowie gesättigt oder ungesättigt sein kann und welcher insbesondere mit ein oder zwei Hydroxylgruppen und/oder einer Aminogruppe substituiert sein kann, wobei auch bis zu 8 nicht benachbarte -CH₂-Gruppen durch -O- ersetzt sein können.

Bezogen auf R⁶ sind bevorzugte Reste C₁₋₁₆- Kohlenwasserstoffreste, insbesondere C₁₋₁₂- Kohlenwasserstoffreste, weiter bevorzugt C₁₋₆- Kohlenwasserstoffreste, am meisten bevorzugt C₁₋₃- Kohlenwasserstoffreste. Vorzugsweise handelt es sich um unverzweigte, acyclische Alkylreste. Sie können auch substituiert sein. Es kann sich z.B. um Mono- oder Dihydroxyalkylreste handeln, die anstelle der Hydroxylgruppen oder zusätzlich auch eine Aminogruppe aufweisen können. Sofern die Kohlenwasserstoffreste durch -O- unterbrochen sind, handelt es sich vorzugsweise um Strukturelemente der Formel -CH₂-CH₂-O- oder -CH₂-CH(CH₃)-O-. Derartige Verbindungen sind in einfacher Weise durch Alkoxylierung der entsprechenden Hydroxylverbindungen zugänglich. Ganz besonders bevorzugte Reste R⁶ sind Methyl-, Ethyl- oder Hydroxymethylreste oder Wasserstoff. Wenn in der 1-Aza-3,7-dioxabicyclo[3.3.0]octan-Verbindung der allgemeinen Formel (I) die Reste R² und R⁴ jeweils für Wasserstoff stehen, dann liegt eine bevorzugte Ausführungsform der Erfindung vor.

Wenn in der 1-Aza-3,7-dioxabicyclo[3.3.0]octan-Verbindung der allgemeinen Formel (I) die Reste R², R⁴, R⁵, R⁷ jeweils für Wasserstoff stehen, und der Rest R⁶ für einen Methyl-, Ethyl- oder Hydroxymethylrest oder Wasserstoff steht, sowie die Reste R¹ und R³ unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt sowie gesättigt oder ungesättigt sein kann, so liegt eine weitere bevorzugte Ausführungsform der Erfindung.

Geeignete Oxazolidine gemäß der allgemeinen Formel (I) sind demnach z.B. 1-Aza-3,7-dioxa-2,8-diheptyl-bicyclo[3.3.0]octan, 1-Aza-3,7-dioxa-2,8-diheptyl-5-methyl-bicyclo[3.3.0]octan, 1-Aza-3,7-dioxa-2,8-diheptyl-5-hydroxymethyl-bicyclo[3.3.0]octan, 1-Aza-3,7-dioxa-2,8-diheptyl-5-ethyl-bicyclo[3.3.0]octan ,1-Aza-3,7-dioxa-2,8-dioctyl-bi-cyclo[3.3.0]octan, 1-Aza-3,7-dioxa-2,8-dioctyl-5-methyl-bicyclo[3.3.0]octan, 1-Aza-3,7-dioxa-2,8-dioctyl-5-hydroxymethyl-bicyclo[3.3.0]octan sowie 1-Aza-3,7-dioxa-2,8-dioctyl-5-ethyl-bicyclo[3.3.0]octan.

Die erfindungsgemäß einsetzbaren Verbindungen der allgemeinen Formel (I) sind insbesondere erhältlich durch Umsetzung von Verbindungen der allgemeinen Formel (II) 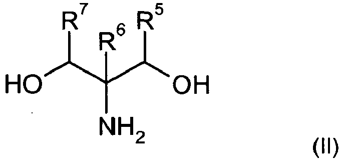 mit Verbindungen der allgemeinen Formeln R¹-C(=O)-R² und R³-C(=O)-R⁴ unter Ringschluss. Eine geeignete Substanz gemäß Formel (II) ist z.B. das 2-Aminopropan-1,3-diol. Für die Reste R¹ bis R⁶ gilt jeweils das zuvor bereits Geschriebene. Die allgemeinen Formeln R¹-C(=O)-R² und R³-C(=O)-R⁴ repräsentieren im Sinne der Erfindung ganz allgemein Riechstoff-Aldehyde oder Riechstoff-Ketone. Riechstoff-Aldehyde sind diejenigen Riechstoffe, welche chemisch ein Aldehyd sind und welche vorteilhafterweise beim Menschen ein insbesondere angenehmes Geruchsempfinden auslösen. Riechstoff-Ketone sind diejenigen Riechstoffe, welche chemisch ein Keton sind und welche vorteilhafterweise beim Menschen ein insbesondere angenehmes Geruchsempfinden auslösen. Besonders geeignete Riechstoff-Aldehyde sowie Riechstoff-Ketone werden weiter oben exemplarisch aufgeführt. Zur Veranschaulichung seien zwei Beispiele gegeben. Beispielsweise steht bei dem Riechstoff-Aldehyd Octanal entsprechend der allgemeinen Formel R¹-C(=O)-R² der Rest R¹ für einen Heptylrest (also für CH₃-(CH₂)₆)- und der Rest R² steht für Wasserstoff oder umgekehrt. Beispielsweise steht bei dem Riechstoff-Keton Methylnonylketon entsprechend der allgemeinen Formel R¹-C(=O)-R² der Rest R¹ für einen Methylrest und der Rest R² steht für einen Nonylrest (also CH₃-(CH₂)₈-) oder umgekehrt.

Als Riechstoff-Aldehyde und/oder Riechstoff-Ketone können grundsätzlich alle üblichen Riechstoff-Aldehyde und/oder Riechstoff-Ketone eingesetzt werden, die insbesondere zur Herbeiführung eines angenehmen Geruchsempfindens beim Menschen eingesetzt werden. Solche Riechstoff-Aldehyde und/oder Riechstoff-Ketone sind dem Fachmann bekannt und auch in der Patentliteratur, beispielsweise in US 2003/0158079 A1, Absätze [0154] und [0155] beschrieben.

Zur Herstellung der erfindungsgemäß einzusetzenden Verbindungen der allgemeinen Formel (I) kann also eine Verbindung der allgemeinen Formel (II) mit Aldehyden, Ketonen oder Mischungen von Ketonen und Aldehyden unter Ringschluss umgesetzt werden. Gemäß einer bevorzugten Ausführungsform der Erfindung leiten sich die Verbindungen der allgemeinen Formel (I) von einem Molekül der allgemeinen Formel (II) und zwei Aldehydmolekülen, welche gleich oder verschieden sein können, oder einem Aldehydmolekül und einem Ketonmolekül ab. Bei der Umsetzung von geringeren als stöchiometrischen Mengen an Aldehyden und/oder Ketonen liegen im Produktgemisch auch monocyclische Verbindungen vor. Der Anteil an bicyclischen Verbindungen zu monocyclischen Verbindungen kann aber in einfacher Weise durch Auswahl des Molverhältnisses zwischen Aldehyd/Keton und der Verbindung der allgemeinen Formel (II) eingestellt werden.

Die Umsetzung wird dabei vorzugsweise in einem geeigneten Lösungsmittel oder in situ durchgeführt. Geeignete Lösungsmittel sind beispielsweise aromatenhaltige Kohlenwasserstoffe wie Toluol. Die Umsetzung wird dabei vorzugsweise bei einer Temperatur im Bereich von 80 bis 150°C, besonders bevorzugt 100 bis 140°C durchgeführt. Beispielsweise wird die Verbindung der allgemeinen Formel (II) unter Stickstoffatmosphäre zusammen mit dem gewünschten Keton und/oder Aldehyd im Lösungsmittel vorgelegt. Sodann wird das Reaktionsgemisch erhitzt. Häufig wird sodann unter Rückfluss am Wasserabscheider erhitzt. Das erhaltene Umsetzungsprodukt wird nach üblichen Verfahren isoliert und gegebenenfalls gereinigt. In WO2007/087977 A1, auf welche hiermit Bezug genommen wird, wird die Herstellung von Verbindungen der allgemeinen Formel (I) auch anhand von Synthesebeispielen detailliert beschrieben.

In einer bevorzugten Ausführungsform der Erfindung wird die 1-Aza-3,7-dioxabicyclo[3.3.0]octan-Verbindung der allgemeinen Formel (I) zusammen mit Riechstoffen eingesetzt, wobei die 1-Aza-3,7-dioxabicyclo[3.3.0]octan-Verbindung in Gewichtsmengen, bezogen auf die Riechstoffgesamtmenge, vorzugsweise im Bereich von 1:100 bis 100:1, insbesondere von 10:1 bis 1:50, eingesetzt wird.

Die weiteren Riechstoffe, die in dem erfindungsgemäßen Verfahren zusätzlich eingesetzt werden können, sind keinen besonderen Beschränkungen unterworfen. So können einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclo-hexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 bis 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Es können auch natürliche Riechstoffgemische eingesetzt werden, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie O-rangenblütenöl, Neroliöl, Orangenschalenöl und Sandelholzöl.

Weitere herkömmliche Riechstoffe, die im Rahmen der vorliegenden Erfindung zusätzlich eingesetzt werden können, sind beispielsweise die ätherischen Öle wie Angelikawurzelöl, Anisöl, Arnikablütenöl, Basilikumöl, Bayöl, Champacablütenöl, Edeltannenöl, Edeltannen-zapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophe-non, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Do-decylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethyl-ether, Isosafrol, Jasmon, Kampfer, Karvakrol, Karvon, p-Kresolmethylether, Cumarin, p-Methoxy-acetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methyl-ether, Nerol, n-Nonylaldehyd, Nonylalkohol, n-Octyl-aldehyd, p-Oxy-Acetophenon, Pentadekanolid, β-Phenyl-ethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Gemäß einer bevorzugten Ausführungsform der Erfindung ist das erfindungsgemäße Verfahren gerichtet auf den Abbau von Fehlgerüchen auf harten und/oder weichen Oberflächen.

Wenn das erfindungsgemäße Verfahren den Abbau von Fehlgerüchen auf Textilien im Rahmen eines Textilbehandlungsverfahrens betrifft, bei welchem das Textil in einem manuellen oder maschinellen Wasch- oder Einweichprozess einer wässrigen Waschflotte ausgesetzt wird, welche ein 1-Aza-3,7-dioxabicyclo[3.3.0]octan-Verbindung der allgemeinen Formel (I) enthält, wobei die Temperatur der Waschflotte 5 bis 95°C, vorzugsweise 10 bis 60°C und insbesondere 15 bis 40°C beträgt, so liegt ebenfalls eine bevorzugte Ausführungsform der Erfindung vor. Dabei wird die 1-Aza-3,7-dioxabi-cyclo[3.3.0]octan-Verbindung der allgemeinen Formel (I) insbesondere als Bestandteil eines, vorzugsweise riechstoffhaltigen, Wasch-, Reinigungs- oder Pflegemittels eingesetzt. Hierbei erfolgt eine besonders gute Verminderung des Schlechtgeruches. Die Behandlungsdauer liegt vorzugsweise in einem Zeitraum von 1 bis 120 Minuten, z.B. im Bereich von 5 bis 60 Minuten, insbesondere von 15 bis 45 Minuten. Die Konzentration der 1-Aza-3,7-dioxabicyclo[3.3.0]octan-Verbindung der allgemeinen Formel (I) in der wässrigen Behandlungsflotte liegt vorzugsweise im Bereich von 0,0004 bis 0,12 g/L, insbesondere von 0,002 bis 0,04 g/L.

Gemäß einer weiteren bevorzugten Ausführungsform der Erfindung betrifft das erfindungsgemäße Verfahren ein Verfahren zum Abbau von Fehlgerüchen auf Textilien, bei welchem eine Flüssigkeit, enthaltend ein 1-Aza-3,7-dioxabicyclo[3.3.0]octan-Verbindung der allgemeinen Formel (I), auf das Textil gesprüht wird. Wenn dabei die Zugabe mittels Spraydose (Druckgasdose, Druckgaspackung, Aerosolpackung) oder mechanisch zu bedienendem Pumpzerstäuber (Pumpspray) erfolgt, unter Bildung eines Sprühnebels, Schaums, einer Paste oder eines Flüssigkeitsstrahls, so liegt wiederum eine bevorzugte Ausführungsform der Erfindung vor.

Das erfindungsgemäße Verfahren eignet sich prinzipiell gleichermaßen zum Fehlgeruchsabbau bei der Textilbehandlung wie bei der Reinigung harter Oberflächen, z.B. Fußboden, sowie beim kosmetischen Einsatz sowie im Air-Care-Bereich und bei der Raumluftverbesserung.

Dementsprechend entspricht ein Verfahren zum Abbau von Fehlgerüchen auf harten Oberflächen, bei welchem die harte Oberfläche mit einer (vorzugsweise wässrigen) Flüssigkeit, enthaltend ein 1-Aza-3,7-dioxabicyclo[3.3.0]octan-Verbindung der allgemeinen Formel (I), in Kontakt gebracht wird, insbesondere durch Abwaschen und/oder durch Einsprühen, einer weiteren bevorzugten Ausführungsform der Erfindung.

Ebenso entspricht ein Verfahren zum Abbau von Fehlgerüchen unter Einsatz einer 1-Aza-3,7-dioxabicyclo[3.3.0]octan-Verbindung der allgemeinen Formel (I) in der Raumluft, insbesondere in Wohnräumen, Küchen, Badezimmern, Toilettenräumen, Schränken sowie Automobilen einer bevorzugten Ausführungsform der Erfindung. Dabei ist insbesondere ein Verfahren bevorzugt, bei welchem die 1-Aza-3,7-dioxabicyclo[3.3.0]octan-Verbindung der allgemeinen Formel (I) über Sticks, Karten, Blöcke oder Spray in die Raumluft eingebracht wird.

Weiterhin ist in diesem Zusammenhang ein Verfahren bevorzugt, bei welchem die Freisetzung der 1-Aza-3,7-dioxabicyclo[3.3.0]octan-Verbindung der allgemeinen Formel (I) über einen Spülreiniger zum Einhängen in das WC-Becken erfolgt.

Generell ist es bevorzugt, das erfindungsgemäße Verfahren so auszuführen, dass die 1-Aza-3,7-dioxabicyclo[3.3.0]octan-Verbindung der allgemeinen Formel (I) zusammen mit Riechstoffen eingesetzt wird. Dementsprechend liegt dann eine bevorzugte Ausführungsform der Erfindung vor, wenn bei einem erfindungsgemäßen Verfahren die 1-Aza-3,7-dioxabicyclo[3.3.0]octan-Verbindung der allgemeinen Formel (I) zusammen mit Riechstoffen eingesetzt wird, wobei die 1-Aza-3,7-dioxabi-cyclo[3.3.0]octan-Verbindung in Gewichtsmengen, bezogen auf die Riechstoffgesamtmenge, vorzugsweise im Bereich von 1:100 bis 100:1, insbesondere von 10:1 bis 1:50, eingesetzt wird.

Erfindungsgemäß kann ein Wasch-, Reinigungs- oder Pflegemittel, enthaltend eine erfindungsgemäß einsetzbare 1-Aza-3,7-dioxabicyclo[3.3.0]octan-Verbindung der allgemeinen Formel (I) vorzugsweise in Mengen von 0,0001 bis 5 Gew.-%, Gew.-% bezogen auf das gesamte Mittel, eingesetzt werden, um Fehlgerüche abzubauen.

Bevorzugt enthalten erfindungsgemäß einsetzbare Wasch-, Reinigungs- oder Pflegemittel (im folgenden Wasch- oder Reinigungsmittel genannt) neben der 1-Aza-3,7-dioxabicyclo[3.3.0]octan-Verbindung der allgemeinen Formel (I) wenigstens eine, vorzugsweise mehrere, aktive Komponenten, insbesondere wasch-, pflege- und/oder reinigungsaktive Komponenten, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen (Gerüststoffe), Bleichmittel, Bleichaktivatoren, Bleichstabilisatoren, Bleichkatalysatoren, Bügelhilfsmittel, Riechstoffe, Einlaufverhinderer, Elektrolyte, Enzyme, Farbschutzstoffe, Farbstoffe, Farbübertragungsinhibitoren, Fluoreszensmittel, Fungizide, Germizide, geruchskomplexierende Substanzen, Hydrotrope, Klarspüler, Komplexbildner, Konservierungsmittel, Korrosionsinhibitoren, optische Aufheller, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungsmittel, Verfärbungsinhibitoren, Vergrauungsinhibitoren, Vitamine und/oder Avivage-Wirkstoffe.

Die Mengen der weiteren möglichen Inhaltsstoffe in den erfindungsgemäß einsetzbaren Waschoder Reinigungsmitteln orientieren sich jeweils am Einsatzzweck der betreffenden Mittel und der Fachmann ist mit den Größenordnungen der einzusetzenden Mengen der optionalen Inhaltsstoffe grundsätzlich vertraut oder kann diese der zugehörigen Fachliteratur entnehmen.

Je nach Einsatzzweck der erfindungsgemäß einsetzbaren Wasch- oder Reinigungsmittel wird man beispielsweise den Tensidgehalt höher oder niedriger wählen. Üblicherweise liegt z.B. der Tensidgehalt beispielsweise von Waschmitteln zwischen z.B. 5 und 50 Gew.-%, vorzugsweise zwischen 10 und 30 Gew.-% und insbesondere zwischen 15 und 25 Gew.-%, während Reinigungsmittel für das maschinelle Geschirrspülen üblicherweise zwischen z.B. 0,1 und 10 Gew.-%, vorzugsweise zwischen 0,5 und 7,5 Gew.-% und insbesondere zwischen 1 und 5 Gew.-% Tenside enthalten.

Die erfindungsgemäß einsetzbaren Wasch- oder Reinigungsmittel können vorzugsweise Tenside enthalten, wobei insbesondere anionische Tenside, nichtionische Tenside und deren Gemische, aber auch kationische Tenside in Frage kommen.

Zu den optional einsetzbaren nichtionischen Tensiden gehören die Alkoxylate, insbesondere die Ethoxylate und/oder Propoxylate, von gesättigten oder ein- bis mehrfach ungesättigten linearen oder verzweigtkettigen Alkoholen mit 10 bis 22 C-Atomen, vorzugsweise 12 bis 18 C-Atomen. Der Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Auch kommen die Ethylenoxid- und/oder Propylenoxid-Insertionsprodukte von Fettsäurealkylestern sowie Fettsäurepolyhydroxyamide, in Betracht.

Zur optionalen Einarbeitung in die erfindungsgemäß einsetzbaren Mittel geeignete sogenannte Alkylpolyglykoside sind Verbindungen der allgemeinen Formel (G)ₙ-OR⁸, in der R⁸ einen Alkyl- oder Alkenylrest mit 8 bis 22 C-Atomen, G eine Glykoseeinheit und n eine Zahl zwischen 1 und 10 bedeuten. Bei der Glykosidkomponente (G)ₙ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil R⁸ der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen R⁸=Dodecyl und R⁸=Tetradecyl.

Nichtionisches Tensid ist in erfindungsgemäß einsetzbaren Wasch- oder Reinigungsmitteln vorzugsweise in Mengen von 0,1 bis 30 Gew.-%, insbesondere von 1 bis 25 Gew.-% optional enthalten, Gew.-% bezogen auf das gesamte Wasch- oder Reinigungsmittel.

Die Wasch- oder Reinigungsmittel können stattdessen oder zusätzlich weitere optionale Tenside enthalten, vorzugsweise Aniontenside.

Vorzugsweise sind Aniontenside des Sulfat- oder Sulfonat-Typs, in Mengen von vorzugsweise nicht über 30 Gew.-%, insbesondere von 0,1 bis 18 Gew.-%, jeweils bezogen auf gesamtes Wasch- oder Reinigungsmittel, optional enthalten. Als für den Einsatz in den erfindungsgemäßen Wasch- oder Reinigungsmittel besonders geeignete Aniontenside sind die Alkyl- und/oder Alkenylsulfate mit 8 bis 22 C-Atomen, die ein Alkali-, Ammonium- oder Alkyl- beziehungsweise Hydroxyalkyl-substituiertes Ammoniumion als Gegenkation tragen, zu nennen. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- beziehungsweise Hydroxyalkyl-substituierten Ammoniumbasen hergestellt werden. Derartige Alkyl- und/oder Alkenylsulfate sind in den Wasch- oder Reinigungsmitteln vorzugsweise in Mengen von 0,1 bis 20 Gew.-%, insbesondere von 0,5 bis 18 Gew.-% optional enthalten.

Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10, Ethylenglykol-Gruppen pro Molekül. Zu den einsetzbaren Aniontensiden vom Sulfonat-Typ gehören die durch Umsetzung von Fettsäureestern mit Schwefeltrioxid und anschließender Neutralisation erhältlichen α-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren.

Besonders bevorzugt optional einsetzbare Aniontenside sind die Alkylbenzolsulfonate, wie z.B. Natrium-Dodecylbenzolsulfonat.

Anionisches Tensid ist in erfindungsgemäß einsetzbaren Wasch- oder Reinigungsmitteln vorzugsweise in Mengen von 0,1 bis 30 Gew.-%, insbesondere von 1 bis 25 Gew.-% optional enthalten, Gew.-% bezogen auf das gesamte Wasch- oder Reinigungsmittel.

Als weitere fakultative tensidische Inhaltsstoffe der erfindungsgemäß einsetzbaren Wasch- oder Reinigungsmittel kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 bis 100 Gew.-% aus gesättigten C₁₂-C₁₈-Fettsäureseifen und zu bis 50 Gew.-% aus Ölsäureseife zusammengesetzt sind. Vorzugsweise ist Seife in dem erfindungsgemäßen Wasch- oder Reinigungsmitteln in Mengen von 0,1 bis 5 Gew.-% optional enthalten. Insbesondere in flüssigen Wasch- oder Reinigungsmitteln können jedoch auch höhere Seifenmengen von bis zu 20 Gew.-% optional enthalten sein.

Auch Kationtenside können optional in den erfindungsgemäß einsetzbaren Wasch- oder Reinigungsmitteln enthalten sein. Beispiele für Kationtenside sind quartäre Ammonium-Verbindungen mit vorzugsweise einem oder insbesondere zwei hydrophoben Alkyl-Resten. Besonders bevorzugt sind Esterquats, also quartäre Ammonium-Verbindungen mit zwei hydrophoben Resten, die jeweils eine Ester-Gruppe als sogenannte Sollbruchstelle für einen leichteren biologischen Abbau enthalten. Bevorzugt einsetzbare Esterquats sind Methyl-N-(2-hydroxyethyl)-N,N-di(talgacyloxy-ethyl)ammonium-methosulfat, Bis-(pal-mitoyloxyethyl)-hydroxyethyl-methyl-ammonium-methosulfat, 1,2-Bis-[talgacyloxy]-3-trimethylammoniumpropanchlorid, N,N-Dimethyl-N,N-di(talgacyloxyethyl)ammonium-methosulfat oder Methyl-N,N-bis(stearoyloxyethyl)-N-(2-hydroxyethyl)ammonium-methosulfat.

Die kationischen Tenside sind in den erfindungsgemäß einsetzbaren Wasch- oder Reinigungsmitteln optional in Mengen von vorzugsweise 0,05 bis 20 Gew.-%, bezogen auf das gesamte Waschoder Reinigungsmittel, enthalten. Mengen von 0,1 bis 5 Gew.-% sind besonders bevorzugt.

Gemäß einer bevorzugten Ausführungsform der Erfindung sind Tenside in erfindungsgemäß einsetzbaren Wasch- oder Reinigungsmitteln in einer Gesamtmenge von vorzugsweise 5 bis 50 Gew.-%, insbesondere von 8 bis 30 Gew.-%, enthalten. Insbesondere in Wäschenachbehandlungsmitteln werden vorzugsweise bis zu 30 Gew.-%, insbesondere 5 bis 15 Gew.-% Tenside, unter diesen bevorzugt wenigstens anteilsweise Kationtenside, eingesetzt.

Ein erfindungsgemäß einsetzbares Wasch- oder Reinigungsmittel kann vorzugsweise mindestens einen Builder, vorzugsweise einen wasserlöslichen und/oder wasserunlöslichen, organischen und/oder anorganischen Builder enthalten. Bevorzugt ist der Einsatz wasserlöslicher Builder.

Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt.

Organische Buildersubstanzen können gewünschtenfalls in Mengen bis zu 40 Gew.-%, insbesondere bis zu 25 Gew.-% und vorzugsweise von 1 bis 8 Gew.-% in den erfindungsgemäßen Waschoder Reinigungsmittel enthalten sein. Mengen nahe der genannten Obergrenze werden vorzugsweise in pastenförmigen oder flüssigen, insbesondere wasserhaltigen, erfindungsgemäßen Wasch- oder Reinigungsmitteln eingesetzt. Erfindungsgemäße Wasch- oder Reinigungsmittel wie Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von z.B. bis zu 50 Gew.-%, vorzugsweise nicht über 40 Gew.-% und in flüssigen Mitteln insbesondere von 1 bis 5 Gew.-%, in den erfindungsgemäßen Wasch- oder Reinigungsmitteln optional eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln optional eingesetzt.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Amorphe Alkalisilikate sind bevorzugt.

Weiterhin ist es im Sinne einer weiteren Ausführungsform bevorzugt, allenfalls geringe Mengen wasserunlöslicher Buildermaterialien (wie z.B. Zeolith) einzusetzen, beispielsweise in Mengen von 0 bis 5 Gew.-%, z.B. 0,1 bis 2 Gew.-%, bezogen auf das gesamte Wasch- oder Reinigungsmittel.

Buildersubstanzen sind in den erfindungsgemäß einsetzbaren Wasch- oder Reinigungsmitteln vorzugsweise in Mengen bis zu 60 Gew.-%, insbesondere von 5 bis 40 Gew.-%, optional enthalten. Erfindungsgemäße Wäschenachbehandlungsmittel, wie z.B. Weichspüler, sind vorzugsweise frei von anorganischem Builder.

Als optional einsetzbare Persauerstoffverbindungen kommen insbesondere organische Persäuren beziehungsweise persaure Salze organischer Säuren, wie Phthalimidpercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt kann Alkalipercarbonat, Alkaliperborat-Monohydrat oder insbesondere in flüssigen Mitteln Wasserstoffperoxid in Form wässriger Lösungen, die 3 bis 10 Gew.-% Wasserstoffperoxid enthalten, eingesetzt werden. Falls ein erfindungsgemäßes Wasch- oder Reinigungsmittel Bleichmittel, wie vorzugsweise Persauerstoffverbindungen, enthält, sind diese in Mengen von vorzugsweise bis zu 50 Gew.-%, insbesondere von 5 bis 30 Gew.-%, vorhanden. Der optionale Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, optional eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- oder iso-NOBS), Carbonsäureanhydride, insbesondere Phthalsäureanhydrid, acylierte mehrwertige Alkohole, insbesondere Triacetin, Ethylenglykoldiacetat, 2,5-Diacet-oxy-2,5-dihydrofuran und Enolester sowie acetyliertes Sorbitol und Mannitol beziehungsweise deren Mischungen , acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, z.B. N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 bis 10 Gew.-%, insbesondere 2 bis 8 Gew.-%, bezogen auf gesamtes Mittel, optional enthalten sein.

Als in den erfindungsgemäß einsetzbaren Wasch- oder Reinigungsmitteln optional einsetzbare Enzyme kommen insbesondere solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäß einsetzbaren Wasch- oder Reinigungsmitteln, vorzugsweise in Mengen nicht über 5 Gew.-%, insbesondere von 0,2 bis 2 Gew.-%, optional enthalten.

Die erfindungsgemäß einsetzbaren Wasch- oder Reinigungsmittel können als optische Aufheller beispielsweise Derivate der Diaminostilbendisulfonsäure beziehungsweise deren Alkalimetallsalze optional enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholinogruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den optional einsetzbaren Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen können auch Gemische aus verschiedenen Schauminhibitoren verwendet werden, zum Beispiel solche aus Silikonen, Paraffinen oder Wachsen. Vorzugsweise sind die optionalen Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die erfindungsgemäß einsetzbaren Wasch- oder Reinigungsmittel optional auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Waschmittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxylgruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure sowie deren Derivate mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die erfindungsgemäß einsetzbaren Wasch- oder Reinigungsmittel können optional auch Farbübertragungsinhibitoren, vorzugsweise in Mengen von 0,1 bis 2 Gew.-%, insbesondere 0,1 bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone, N-Vinyl-imidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren.

Die optional einsetzbaren Vergrauungsinhibitoren haben das Vermögen, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das erfindungsgemäß einsetzbare Wasch- oder Reinigungsmittel, als optionale Vergrauungsinhibitoren eingesetzt werden.

Zu den in den erfindungsgemäß einsetzbaren Wasch- oder Reinigungsmitteln, insbesondere wenn sie in flüssiger oder pastöser Form vorliegen, optional verwendbaren organischen Lösungsmitteln gehören vorzugsweise Alkohole mit 1 bis 4 C-Atomen, insbesondere Methanol, Ethanol, Isopropanol und tert-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel können in den erfindungsgemäß einsetzbaren Wasch- oder Reinigungsmitteln vorzugsweise in Mengen von nicht über 30 Gew.-%, insbesondere von 6 bis 20 Gew.-%, optional vorhanden sein.

Zur Einstellung eines gewünschten, sich durch die Mischung der übrigen Komponenten nicht von selbst ergebenden pH-Werts können die erfindungsgemäß einsetzbaren Wasch- oder Reinigungsmittel Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, optional enthalten. Derartige pH-Regulatoren können in den erfindungsgemäß einsetzbaren Wasch- oder Reinigungsmitteln vorzugsweise nicht über 20 Gew.-%, insbesondere von 1,2 bis 17 Gew.-%, optional enthalten sein.

Die Herstellung fester erfindungsgemäß einsetzbarer Wasch- oder Reinigungsmittel kann im Prinzip in bekannter Weise, zum Beispiel durch Sprühtrocknen oder Granulation, erfolgen, wobei z.B. optionale Persauerstoffverbindung und optionaler Bleichkatalysator gegebenenfalls später zugesetzt werden. Zur Herstellung erfindungsgemäß einsetzbarer Wasch- oder Reinigungsmittel mit erhöhtem Schüttgewicht, insbesondere im Bereich von 650 bis 950 g/L, ist ein einen Extrusionsschritt aufweisendes Verfahren bevorzugt. Die Herstellung flüssiger erfindungsgemäß einsetzbarer Wasch- oder Reinigungsmittel kann ebenfalls in an sich bekannter Weise erfolgen.

Ein bevorzugtes erfindungsgemäß einsetzbares Wasch- oder Reinigungsmittel ist ein festes, insbesondere pulverförmiges Waschmittel, das neben der 1-Aza-3,7-dioxabicyclo[3.3.0]octan-Verbindung der allgemeinen Formel (I) vorzugsweise Komponenten enthalten kann, die vorzugsweise ausgewählt sind aus den folgenden:
(a) Aniontenside, wie vorzugsweise Alkylbenzolsulfonat, Alkylsulfat, z.B. in Mengen von vorzugsweise 5 bis 30 Gew.-%
(b) Nichtionische Tenside, wie vorzugsweise Fettalkoholpolyglycolether, Alkylpolyglucosid, Fettsäureglucamid z.B. in Mengen von vorzugsweise 0,5 bis 15 Gew.-%
(c) Gerüststoffe, wie z.B. Polycarboxylat, Natriumcitrat, in Mengen von z.B. 0 bis 70 Gew.-%, vorteilhafterweise 5 bis 60 Gew.-%, vorzugsweise 10 bis 55 Gew.-%, insbesondere 15 bis 40 Gew.-%,
(d) Alkalien, wie z.B. Natriumcarbonat, in Mengen von z.B. 0 bis 35 Gew.-% vorteilhafterweise 1 bis 30 Gew.-%, vorzugsweise 2 bis 25 Gew.-%, insbesondere 5 bis 20 Gew.-%,
(e) Bleichmittel, wie z.B. Natriumperborat oder Natriumpercarbonat, in Mengen von z.B. 0 bis 30 Gew.-% vorteilhafterweise 5 bis 25 Gew.-%, vorzugsweise 10 bis 20 Gew.-%,
(f) Korrosionsinhibitoren, z.B. Natriumsilicat, in Mengen von z.B. 0 bis 10 Gew.-%, vorteilhafterweise 1 bis 6 Gew.-%, vorzugsweise 2 bis 5 Gew.-%, insbesondere 3 bis 4 Gew.-%,
(g) Stabilisatoren, z.B. Phosphonate, vorteilhafterweise 0 bis 1 Gew.-%,
(h) Schauminhibitor, z.B. Seife, Siliconöle, Paraffine vorteilhafterweise 0 bis 4 Gew.-%, vorzugsweise 0,1 bis 3 Gew.-%, insbesondere 0,2 bis 1 Gew.-%,
(i) Enzyme, z.B. Proteasen, Amylasen, Cellulasen, Lipasen, vorteilhafterweise 0 bis 2 Gew.-%, vorzugsweise 0,2 bis 1 Gew.-%, insbesondere 0,3 bis 0,8 Gew.-%,
(j) Vergrauungsinhibitor, z.B. Carboxymethylcellulose, vorteilhafterweise 0 bis 1 Gew.-%,
(k) Verfärbungsinhibitor, z.B. Polyvinylpyrrolidon-Derivate, z.B. 0 bis 2 Gew.-%,
(l) Stellmittel, z.B. Natriumsulfat, vorteilhafterweise 0 bis 20 Gew.-%,
(m) Optische Aufheller, z.B. Stilben-Derivat, Biphenyl-Derivat, vorteilhafterweise 0 bis 0,4 Gew.-%, insbesondere 0,1 bis 0,3 Gew.-%,
(n) ggf. weitere Riechstoffe
(o) ggf. Wasser
(p) ggf. Seife
(q) ggf. Bleichaktivatoren
(r) ggf. Cellulosderivate
(s) ggf. Schmutzabweiser,
Gew.-% jeweils bezogen auf das gesamte Mittel.

In einer weiter bevorzugten Ausführungsform ist das erfindungsgemäß einsetzbare Wasch- oder Reinigungsmittel festförmig, insbesondere teilchenförmig und enthält neben der 1-Aza-3,7-dioxabicyclo[3.3.0]octan-Verbindung der allgemeinen Formel (I) noch 5 bis 55 Gew.-% Gerüststoffe, 2,5 bis 20 Gew.-% Aniontensid, 1 bis 20 Gew.-% Niotensid, 1 bis 25 Gew.-% Bleichmittel, 0,5 bis 8 Gew.-% Bleichaktivator und 0,1 bis 40 Gew.-% Stellmittel, insbesondere Alkalisulfat, sowie bis zu 2 Gew.-%, insbesondere 0,4 bis 1,2 Gew.-% Enzym, vorzugsweise teilchenförmig konfektioniertes Enzym, insbesondere Protease, Lipase, Amylase, Cellulase und/oder Oxidoreduktase. Diese Ausführungsform kann optional auch frei von Bleichmittel und Bleichaktivator sein.

In einer anderen bevorzugten Ausführungsform der Erfindung liegt das erfindungsgemäß einsetzbare Wasch- oder Reinigungsmittel, enthaltend eine 1-Aza-3,7-dioxabicyclo[3.3.0]octan-Verbindung der allgemeinen Formel (I), in flüssiger Form vor, vorzugsweise in Gelform. Bevorzugte erfindungsgemäß einsetzbare flüssige Wasch- oder Reinigungsmittel haben Wassergehalte von z.B. 10 bis 95 Gew.-%, vorzugsweise 20 bis 80 Gew.-% und insbesondere 30 bis 70 Gew.-%, bezogen auf das gesamte Mittel. Im Falle von flüssigen Konzentraten kann der Wassergehalt auch besonders gering sein, z.B. ≤ 30 Gew.-%, vorzugsweise ≤ 20 Gew.-%, insbesondere ≤ 15 Gew.-%, wie z.B. 0,1 bis 10 Gew.-%, betragen, Gew.-% jeweils bezogen auf das gesamte Mittel. Die flüssigen Verbrauchsprodukte können auch nichtwässrige Lösungsmittel enthalten.

Ein bevorzugtes erfindungsgemäß einsetzbares Wasch- oder Reinigungsmittel ist ein flüssiges, insbesondere gelförmiges Waschmittel, das neben der der 1-Aza-3,7-dioxabicyclo[3.3.0]octan-Verbindung der allgemeinen Formel (I) vorzugsweise Komponenten enthalten kann, die vorzugsweise ausgewählt sind aus den folgenden:
- Aniontenside, wie vorzugsweise Alkylbenzolsulfonat, Alkylsulfat, z.B. in Mengen von vorzugsweise 5 bis 40 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 25 Gew.-%
- Gerüststoffe, wie z.B. Polycarboxylat, Natriumcitrat, vorteilhafterweise 0 bis 25 Gew.-%, vorzugsweise 0,01 bis 10 Gew.-%, insbesondere 0,1 bis 5 Gew.-%,
- Schauminhibitor, z.B. Siliconöle, Paraffine, in Mengen von z.B. 0 bis 10 Gew.-%, vorteilhafterweise 0,1 bis 4 Gew.-%, vorzugsweise 0,2 bis 2 Gew.-%, insbesondere 1 bis 3 Gew.-%,
- Enzyme, z.B. Proteasen, Amylasen, Cellulasen, Lipasen, in Mengen von z.B. 0 bis 3 Gew.-%, vorteilhafterweise 0,1 bis 2 Gew.-%, vorzugsweise 0,2 bis 1 Gew.-%, insbesondere 0,3 bis 0,8 Gew.-%,
- Optische Aufheller, z.B. Stilben-Derivat, Biphenyl-Derivat, in Mengen von z.B. 0 bis 1 Gew.-%, vorteilhafterweise 0,1 bis 0,3 Gew.-%, insbesondere 0,1 bis 0,4 Gew.-%,
- ggf. weitere Riechstoffe
- Wasser
- ggf. Seife, in Mengen von z.B. 0 bis 25 Gew.-%, vorteilhafterweise 1 bis 20 Gew.-%, vorzugsweise 2 bis 15 Gew.-%, insbesondere 5 bis 10 Gew.-%,
- ggf. Lösungsmittel (vorzugsweise Alkohole), vorteilhafterweise 0 bis 25 Gew.-%, vorzugsweise 1 bis 20 Gew.-%, insbesondere 2 bis 15 Gew.-%, Gew.-% jeweils bezogen auf das gesamte Mittel.

Ein besonders bevorzugtes erfindungsgemäß einsetzbares flüssiges Wasch- oder Reinigungsmittel enthält dabei neben der der 1-Aza-3,7-dioxabicyclo[3.3.0]octan-Verbindung der allgemeinen Formel (I) zumindest Aniontenside in Mengen von 0,5 bis 20 Gew.-%, nichtionische Tenside in Mengen von 1 bis 25 Gew.-%, Gerüststoffe in Mengen von 1 bis 25 Gew.-%, Enzyme sowie Wasser. Ein weiteres bevorzugtes erfindungsgemäß einsetzbares Wasch- oder Reinigungsmittel ist ein flüssiger Weichspüler, der neben der der 1-Aza-3,7-dioxabicyclo[3.3.0]octan-Verbindung der allgemeinen Formel (I) vorzugsweise Komponenten enthalten kann, die ausgewählt sind aus den folgenden:
- Kationische Tenside, wie insbesondere Esterquats, z.B. in Mengen von 5 bis 30 Gew.-%,
- Cotenside, wie insbesondere Glycerolmonostearat, Stearinsäure, Fettalkohole und/oder Fettalkoholethoxylate, z.B. in Mengen von 0 bis 5 Gew.-%, vorzugsweise 0,1 bis 4 Gew.-%,
- Emulgatoren, wie insbesondere Fettaminethoxylate, z.B. in Mengen von 0 bis 4 Gew.-%, vorzugsweise 0,1 bis 3 Gew.-%,
- ggf. weitere Riechstoffe
- ggf. Farbstoffe, vorzugsweise im ppm-Bereich
- Lösemittel, wie insbesondere Wasser, z.B. in Mengen von 60 bis 90 Gew.-%,
Gew.-% jeweils bezogen auf das gesamte Mittel.

Ein weiterer Gegenstand der Erfindung ist ein Verfahren zum Abbau von Fehlgerüchen auf Textilien im Rahmen eines Textilbehandlungsverfahrens, bei welchem das Textil in einem manuellen oder maschinellen Wasch- oder Einweichprozess unter Einsatz eines Wasch-, Reinigungs- oder Pflegemittels, enthaltend eine erfindungsgemäß einsetzbare 1-Aza-3,7-dioxabicyclo[3.3.0]octan-Verbindung der allgemeinen Formel (I) vorzugsweise in Mengen von 0,0001 bis 5 Gew.-%, bezogen auf das gesamte Mittel, einer Waschflotte ausgesetzt wird, wobei die Temperatur der Waschflotte 5 bis 95°C, vorzugsweise 10 bis 60°C und insbesondere 15 bis 40°C beträgt.

### Beispiele

### Beispiel 1)

Ein Versuchsbehälter von 35 L Rauminhalt war mit einer 1 cm großen Öffnung zum Anschluss eines Schlauchs versehen, mit dem eine olfaktorische Kontrolle der darin befindlichen Atmosphäre stattfinden konnte. Über eine seitlich Klappe in Bodennähe konnte der Behälter rasch geöffnet und mit Untersuchungsmedien bestückt werden. Der Versuch fand bei Raumtemperatur unter Normaldruck statt. Es wurden zwei identisch aufgebaute Behälter eingesetzt. In die gereinigten und geruchsneutralen Behälter wurde eine Petri-Schale mit 5 mL einer 10%igen Fehlgeruchslösung A (in Polyethylenglykol 2000) platziert. Diese Fehlgeruchslösung A (Rezeptur siehe unten) dient zur Nachstellung eines Fehlgeruches, welcher für Toilettenräume üblich ist. Nach einer Stunde wurde die Petri Schale mit dem Standard entfernt. Nach 30 min ergab die olfaktorische Kontrolle der beiden Behälter jeweils einen intensiven Geruch nach dem Toiletten-Fehlgeruch. Mittels einer Sprühflasche wurde eine Prüflösung [siehe unten: Methylserinollösung plus Aldehyd C10 sowie Oxazolidinlösung (bicyclisches Oxazolidin gebildet durch Reaktion von Aldehyd C10 mit Methylserinol)] fein verteilt in einen der Versuchsbehälter eingebracht. Dabei wurde jeweils ein Sprühstoß mit einem Volumen von 0,1 mL erzeugt. Als Lösungsmittel wurde Ethanol verwendet. Die Lösungen hatten einen 10%igen Aktivstoffgehalt. Die Prüflösungen unterschieden sich also nur insoweit, dass bei der Oxazolidinlösung das Reaktionsprodukt aus Reaktion von Aldehyd C10 mit Methylserinol, also das Oxazolidin vorlag, während bei der Methylserinollösung plus Aldehyd C10 der Aminoalkohol und der Riechstoff-Aldehyd separat vorlagen. Unmittelbar nach dem Sprühstoß sowie in den in der Tabelle aufgeführten Zeitabständen wurde eine Beurteilung der Geruchsintensität des Toilettenfehlgeruches in der Kammer durchgeführt. Die Skala der Geruchsintensität reicht von stark (Wert 10) bis kaum wahrnehmbar (Wert 1). Die Beurteilung wurde von geruchlich geschulten Menschen vorgenommen.

Ergebnisse:

| | Intensität nach 0 h | Intensität nach 1 h | Intensität nach 2 h | Intensität nach 4 h | Intensität nach 8 h |
|---|---|---|---|---|---|
| Kammer ohne weitere Beaufschlagung | 10 | 6 | 6 | 5 | 4 |
| Kammer beaufschlagt mit Methylserinol-lösung plus Aldehyd C10 | 10 | 4 | 2 | 1 | 0 |
| Kammer beaufschlagt mit Oxazolidin-lösung | 10 | 3 | 1 | 0 | 0 |

Wie die Versuchsergebnisse zeigen, führte die Oxazolidinlösung bereits nach 4 h zu einer vollständigen Auslöschung des Fehlgeruches. Im Falle der Methylserinollösung (Methylserinollösung plus Aldehyd C10) konnte die Geruchsauslöschung erst nach der doppelten Zeit, also nach 8 h erzielt werden.

Die Zugabe von Oxazolidinen in das System ermöglichte insgesamt eine effiziente Auslöschung von Fehlgeruch.

### Fehlgeruchlösung A (Toilette)

| | |
|---|---|
| 3-Methylbutansäure | 20 Gew.-% |
| n-Butansäure | 20 Gew.-% |
| n-Hexansäure | 20 Gew.-% |
| 2,3-Benzopyrrol | 20 Gew.-% |
| 4-Methyl-2,3-Benzopyrrol | 10 Gew.-% |
| Ammoniaklösung (25%ig in Wasser) | 10 Gew.-% |

### Beispiel 2)

Zur Evaluierung wurden unterschiedliche Fehlgeruch-Standards für Schlechtgerüche herangezogen. Standard Schweiß (Fehlgeruchslösung B (Rezeptur siehe unten) dient zur Nachstellung eines Schweiß-Fehlgeruches) repräsentierte einen Schweißgeruch, der von Menschen ausgeht, Standard Toilette (Fehlgeruchslösung A, siehe oben) repräsentierte den schlechten Geruch in Toilettenräumen und Standard Moder (Fehlgeruchslösung C (Rezeptur siehe unten)) repräsentierte einen Modergeruch, wie er für schlecht belüftete Kellerräume typisch ist.

In einem Schraubdeckelglas mit definiertem Volumen von 2 L wurde jeweils ein mit Fehlgeruch (Standard Schweiß, Toilette oder Moder) ausgerüstetes Textil im Format 10 x 10 cm mit einem Klettklebeband am Schraubdeckel fixiert. Jedes Textil wurde dazu jeweils mit 38 mg des betreffenden Fehlgeruch-Standards beaufschlagt. Das beaufschlagte Textil wurde dann ins Schraubdeckelglas eingebracht und reifte dann dort für 2 h bei geschlossenem Deckel. Danach wurden jeweils 35 g der Prüflösung eingebracht [Serinol- oder Methylserinollösung plus Aldehyd C10 sowie Oxazolidinlösung (bicyclisches Oxazolidin gebildet durch Reaktion von Aldehyd C10 mit Serinol oder Methylserinol)]. Die Prüflösungen wurden als molare Lösungen in Ethanol erstellt. Die Prüflösungen unterschieden sich somit wiederum nur insoweit, dass im Falle der Oxazolidinlösungen das Reaktionsprodukt aus Riechstoff-Aldehyd und Aminoalkohol, also das Oxazolidin vorlag, während in den Vergleichsfällen der Aminoalkohol und der Riechstoff-Aldehyd separat vorlagen. Die Beurteilung des Geruches erfolgte danach über einen Zeitraum von 24 Stunden durch geruchlich geschulte Menschen. Als Textilien kamen Polyester und Baumwolle zum Einsatz (siehe unten).

### Ergebnisse:

### Standard Schweiß auf Polyester

| | Intensität nach 0 h | Intensität nach 2 h | Intensität nach 4 h | Intensität nach 6 h | Intensität nach 24 h |
|---|---|---|---|---|---|
| Standard Schweiß | 10 | 9 | 7 | 6 | 3 |
| Oxazolidin aus Serinol und Aldehyd C10 | 10 | 3 | 2 | 1 | 0 |
| Aldehyd C10 plus Serinol | 10 | 3 | 3 | 2 | 1 |

### Standard Toilette auf Polyester

| | Intensität nach 0 h | Intensität nach 2 h | Intensität nach 4 h | Intensität nach 6 h | Intensität nach 24 h |
|---|---|---|---|---|---|
| Standard Toilette | 10 | 9 | 8 | 8 | 7 |
| Oxazolidin aus Serinol und Aldehyd C10 | 10 | 6 | 4 | 3 | 3 |
| Aldehyd C10 plus Serinol | 10 | 7 | 6 | 4 | 4 |

### Standard Schweiß auf Baumwolle

| | Intensität nach 0 h | Intensität nach 2 h | Intensität nach 4 h | Intensität nach 6 h | Intensität nach 24 h |
|---|---|---|---|---|---|
| Standard Schweiß | 10 | 9 | 5 | 4 | 2 |
| Oxazolidin aus Methylserinol und Aldehyd C10 | 10 | 3 | 1 | 1 | 0 |
| Aldehyd C10 plus Methylserinol | 10 | 3 | 3 | 2 | 1 |

### Standard Moder auf Polyester

| | Intensität nach 0 h | Intensität nach 2 h | Intensität nach 4 h | Intensität nach 6 h | Intensität nach 24 h |
|---|---|---|---|---|---|
| Standard Moder | 10 | 9 | 8 | 8 | 3 |
| Oxazolidin aus Serinol und Aldehyd C10 | 10 | 4 | 2 | 1 | 0 |
| Aldehyd C10 plus Serinol | 10 | 5 | 4 | 3 | 2 |

Auch hier zeigen alle Beispiele die überlegene Wirkung der Oxazolidine bei der Fehlgeruchsbekämpfung im Vergleich zu der Kombination aus Riechstoff-Aldehyd und Aminoalkohol.

### Fehlgeruchlösung B (Schweiss)

| | |
|---|---|
| Octansäure | 20 Gew.-% |
| Nonansäure | 20 Gew.-% |
| 3-Methylbutansäure | 20 Gew.-% |
| 2-Ethyl-2-Hexensäure | 20 Gew.-% |
| 3-Mercapto-1-Hexanol | 20 Gew.-% |

### Fehlgeruchslösung C (Moder)

| | |
|---|---|
| Patchouly (Essential Oil) | 10 Gew.-% |
| Geosmin: 0,1% in DPG | 10 Gew.-% |
| Fixolide | 10 Gew.-% |
| Terpineol-4 | 10 Gew.-% |
| n-Hexansäure | 20 Gew.-% |
| 3-Methylbutansäure | 20 Gew.-% |
| 4-Ethyloctansäure | 20 Gew.-% |

Fixolide: (+)-1-[(6S)-3,5,5,6,8,8-hexamethyl-5,6,7,8-tetrahydronaphthalen-2-yl]ethanon Geosmin: (4*S*,4a*S*,8a*R*)-4,8a-Dimethyl-1,2,3,4,5,6,7,8-octahydronaphthalin-4a-ol

## Patentansprüche

1. Verfahren zum Abbau von Fehlgerüchen durch Einsatz einer 1-Aza-3,7-dioxabicyclo[3.3.0]octan-Verbindung der allgemeinen Formel (I) 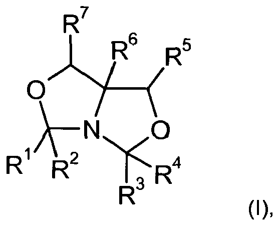 wobei
R¹, R², R³, R⁴ unabhängig voneinander für Reste stehen, die in einer Verbindung der allgemeinen Formel R¹-C(=O)-R² oder R³-C(=O)-R⁴ einen Riechstoff-Aldehyd mit mindestens 6 Kohlenstoffatomen oder ein Riechstoff-Keton mit mindestens 6 Kohlenstoffatomen ergeben,
R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann.

2. Verfahren gemäß Anspruch 1, **dadurch gekennzeichnet, dass** der Riechstoff-Aldehyd ausgewählt ist aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxy-benzaldehyd, Benzaldehyd, 3-(4-tert-Butylphenyl)-propanal, 2-Methyl-3-(para-methoxyphenyl)-propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhy-drozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methyl-phenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethyl-cyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pen-tenyl)-3-cyclohexen-1-carboxaldehyd, 5- oder 6-Methoxyhexahydro-4,7-methanindan-1-oder-2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Di-methyl-4,8-decadienal, Päonienaldehyd (6,10-Dimethyl-3-oxa-5,9-unde-cadien-1-al), Hexahydro- 4,7-methanindan-1-carboxaldehyd, 2-Methyloctanal, alpha-Methyl-4-(1-methylethyl)benzolace-taldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

3. Verfahren gemäß Anspruch 1 oder 2, **dadurch gekennzeichnet, dass** das Riechstoff-Keton ausgewählt ist aus Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-Ionon, beta-Ionon, gamma-Methyl-Ionon, Fleuramon (2-heptylcyclopentanon), Dihydrojasmon, cis-Jasmon, iso-E-Super (1-(1,2,3,4,5,6,7,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldecahydro-2-naphton, Dimethyloctenon, Frescomenthe (2-butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-meth-oxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-benzodioxol-5-yl) butan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amyl-cyclohexanon), 4-tert-butyl cyclohexanon, Delphon (2-pentyl-cyclopentanon), Muscon (CAS 541-91-3), Neobutenon (1-(5,5-dimethyl-1-cyclohexenyl)pent-4-en-1-on), Plicaton (CAS 41724-19-0), Velouton (2,2,5-trimethyl-5-pentyl-cyclopentan-1-on), 2,4,4,7-Tetramethyl-oct-6-en-3-on sowie Tetrameran (6,10-dimethylundecen-2-on).

4. Verfahren nach einem der Ansprüche 1 bis 3, **dadurch gekennzeichnet, dass** in der 1-Aza-3,7-dioxabicyclo[3.3.0]octan-Verbindung der allgemeinen Formel (I) die Reste R⁵ und R⁷ unabhängig voneinander jeweils für Wasserstoff oder einen C₁₋₆-Kohlenwasserstoffrest, der ggf. substituiert sein kann, vorzugsweise C₁₋₃- Kohlenwasserstoffrest stehen, insbesondere sind R⁵ und R⁷ jeweils Wasserstoff oder jeweils ein Methyl- oder Ethylrest.

5. Verfahren nach einem der Ansprüche 1 bis 4, **dadurch gekennzeichnet, dass** in der 1-Aza-3,7-dioxabicyclo[3.3.0]octan-Verbindung der allgemeinen Formel (I) der Rest R⁶ für einen Methyl-, Ethyl- oder Hydroxymethylrest oder Wasserstoff steht.

6. Verfahren nach einem der Ansprüche 1 bis 5, **dadurch gekennzeichnet, dass** in der 1-Aza-3,7-dioxabicyclo[3.3.0]octan-Verbindung der allgemeinen Formel (I) die Reste R² und R⁴ jeweils für Wasserstoff stehen.

7. Verfahren nach einem der Ansprüche 1 bis 6, **dadurch gekennzeichnet, dass** in der 1-Aza-3,7-dioxabicyclo[3.3.0]octan-Verbindung der allgemeinen Formel (I) die Reste R², R⁴, R⁵, R⁷ jeweils für Wasserstoff stehen, und der Rest R⁶ für einen Methyl-, Ethyl- oder Hydroxymethylrest oder Wasserstoff steht, sowie dass die Reste R¹ und R³ unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt sowie gesättigt oder ungesättigt sein kann.

8. Verfahren gemäß einem der Ansprüche 1 bis 7 zum Abbau von Fehlgerüchen auf harten und/oder weichen Oberflächen.

9. Verfahren gemäß einem der Ansprüche 1 bis 8 zum Abbau von Fehlgerüchen auf weichen O-berflächen, insbesondere Textilien, im Rahmen eines Textilbehandlungsverfahrens, bei welchem das Textil in einem manuellen oder maschinellen Wasch- oder Einweichprozess einer Waschflotte ausgesetzt wird, welche eine 1-Aza-3,7-dioxabicyclo[3.3.0]octan-Verbindung der allgemeinen Formel (I) enthält, wobei die Temperatur der Waschflotte 5 bis 95°C, vorzugsweise 10 bis 60°C und insbesondere 15 bis 40°C beträgt.

10. Verfahren gemäß einem der Ansprüche 1 bis 9 zum Abbau von Fehlgerüchen auf Textilien bei welchem eine Flüssigkeit, enthaltend eine 1-Aza-3,7-dioxabicyclo[3.3.0]octan-Verbindung der allgemeinen Formel (I), auf das Textil gesprüht wird.

11. Verfahren gemäß einem der Ansprüche 1 bis 10, bei welchem die 1-Aza-3,7-dioxabicyclo[3.3.0]octan-Verbindung der allgemeinen Formel (I) zusammen mit Riechstoffen eingesetzt wird, wobei die 1-Aza-3,7-dioxabicyclo[3.3.0]octan-Verbindung in Gewichtsmengen, bezogen auf die Riechstoffgesamtmenge, vorzugsweise im Bereich von 1:100 bis 100:1, insbesondere von 10:1 bis 1:50, eingesetzt wird.

12. Verfahren gemäß einem der Ansprüche 1 bis 11 zum Abbau von Fehlgerüchen auf harten O-berflächen, bei welchem die harte Oberfläche mit einer Flüssigkeit, enthaltend eine 1-Aza-3,7-dioxabicyclo[3.3.0]octan-Verbindung der allgemeinen Formel (I), in Kontakt gebracht wird, insbesondere durch Abwaschen und/oder durch Einsprühen.

13. Verfahren gemäß einem der Ansprüche 1 bis 12 zum Abbau von Fehlgerüchen in der Raumluft, insbesondere in Wohnräumen, Küchen, Badezimmern, Toilettenräumen, Schränken sowie Automobilen, wobei die 1-Aza-3,7-dioxabicyclo[3.3.0]octan-Verbindung der allgemeinen Formel (I) insbesondere über Sticks, Karten, Blöcke oder Spray in die Raumluft eingebracht wird.

## Claims

1. A method for degrading off-odors by employing a 1-aza-3,7-dioxabicyclo[3.3.0]octane compound of the general formula (I) 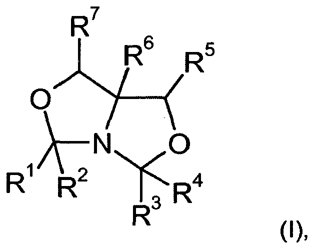 wherein
R¹, R², R³, R⁴ mutually independently denote residues that, in a compound of the general formula R¹-C(=O)-R² or R³-C(=O)-R⁴, yield a fragrance aldehyde having at least six carbon atoms or a fragrance ketone having at least six carbon atoms,
R⁵, R⁶, R⁷ mutually independently denote H or a hydrocarbon residue that can be acyclic or cyclic, substituted or unsubstituted, branched or unbranched as well as saturated or unsaturated.

2. The method according to Claim 1, **characterized in that** the fragrance aldehyde is selected from adoxal (2,6,10-trimethyl-9-undecenal), anisaldehyde (4-methoxybenzaldehyde), cymal (3-(4-isopropylphenyl)-2-methylpropanal), ethyl vanillin, florhydral (3-(3-isopropylphenyl)butanal), helional (3-(3,4-methylenedioxyphenyl)-2-methylpropanal), heliotropin, hydroxycitronellal, lauraldehyde, lyral (3- and 4-(4-hydroxy-4-methyl pentyl)-3-cyclohexene-1-carboxaldehyde), methyl nonyl acetaldehyde, lilial (3-(4-tert-butylphenyl)-2-methylpropanal), phenyl acetaldehyde, undecylenealdehyde, vanillin, 2,6,10-trimethyl-9-undecenal, 3-dodecen-1-al, alpha-n-amylcinnamaldehyde, melonal (2,6-dimethyl-5-heptenal), 2,4-dimethyl-3-cyclohexene-1-carboxaldehyde (triplal), 4-methoxybenzaldehyde, benzaldehyde, 3-(4-tert-butylphenyl)propanal, 2-methyl-3-(para-methoxyphenyl)propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decylidene-8)-butanal, octahydro-4,7-methane-1H-indenecarboxaldehyde, 3-ethoxy-4-hydroxybenzaldehyde, para-ethyl-alpha,alpha-dimethylhydrocinnamaldehyde, alpha-methyl-3,4-(methylenedioxy)hydrocinnamaldehyde, 3,4-methylenedioxybenzaldehyde, alpha-n-hexylcinnamaldehyde, m-cymene-7-carboxaldehyde, alpha-methyl phenyl acetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexene-3-carboxaldehyde, 4-(4-hydroxy-4-methyl pentyl)-3-cylohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methane indane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyloctanal, trans-4-decenal, 2,6-nonadienal, para-tolylacetaldehyde, 4-methyl phenyl acetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methane indane-1-carboxaldehyde, 2-methyloctanal, alpha-methyl-4-(1-methyl ethyl)benzeneacetaldehyde, 6,6-dimethyl-2-norpinene-2-propionaldehyde, para-methyl phenoxy acetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]-hept-5-ene-2-carbaldehyde, 9-decenal, 3-methyl-5-phenyl-1-pentanal, methyl nonyl acetaldehyde, hexanal, and trans-2-hexenal.

3. The method according to Claim 1 or 2, **characterized in that** the fragrance ketone is selected from methyl beta-naphthyl ketone, musk indanone (1,2,3,5,6,7-hexahydro-1,1,2,3,3-pentamethyl-4H-inden-4-one), tonalide (6-acetyl-1,1,2,4,4,7-hexamethyltetraline), alpha-damascone, beta-damascone, delta-damascone, isodamascone, damascenone, methyl dihydrojasmonate, menthone, carvone, camphor, koavone (3,4,5,6,6-pentamethylhept-3-en-2-one), fenchone, alpha-ionone, beta-ionone, gamma-methyl ionone, fleuramone (2-heptylcyclopentanone), dihydrojasmone, cis-jasmon, iso-E-super (1-(1,2,3,4,5,6,7,8-octahydro-2,3,8,8-tetramethyl-2-naphthalenyl)-ethan-1-one (and isomers)), methyl cedrenyl ketone, acetophenone, methyl acetophenone, para-methoxyacetophenone, methyl beta-naphthyl ketone, benzylacetone, benzophenone, para-hydroxyphenyl butanone, celery ketone (3-methyl-5-propyl-2-cyclohexenone), 6-isopropyldecahydro-2-naphthone, dimethyl octenone, Frescomenthe (2-butan-2-yl-cyclohexan-1-one), 4-(1-ethoxyvinyl)-3,3,5,5-tetramethylcyclohexanone, methyl heptenone, 2-(2-(4-methyl-3-cyclohexen-1-yl)propyl)cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)-indanone, 4-damascol, dulcinyl (4-(1,3-benzodioxol-5-yl)butan-2-one), hexalone (1-(2,6,6-trimethyl-2-cyclohexen-1-yl)-1,6-heptadien-3-one), isocyclemone E (2-acetonaphthone-1,2,3,4,5,6,7,8-octahydro-2,3,8,8-tetramethyl), methyl nonyl ketone, methyl cyclocitrone, methyl lavender ketone, orivone (4-tert-amyl cyclohexanone), 4-tert-butyl cyclohexanone, delphone (2-pentylcyclopentanone), muscone (CAS 541-91-3), neobutenone (1-(5,5-dimethyl-1-cyclohexenyl)pent-4-en-1-one), plicatone (CAS 41724-19-0), veloutone (2,2,5-trimethyl-5-pentylcyclopentan-1-one), 2,4,4,7-tetramethyloct-6-en-3-one, and tetrameran (6, 10-dimethylundecen-2-one).

4. The method according to one of Claims 1 to 3, **characterized in that** in the 1-aza-3,7-dioxabicyclo[3.3.0]octane compound of the general formula (I), the residues R⁵ and R⁷ mutually independently each denote hydrogen or a C₁₋₆ hydrocarbon residue that can optionally be substituted, preferably a C₁₋₃ hydrocarbon residue, in particular R⁵ and R⁷ are each hydrogen or each a methyl or ethyl residue.

5. The method according to one of Claims 1 to 4, **characterized in that** in the 1-aza-3,7-dioxabicyclo[3.3.0]octane compound of the general formula (I), the residue R⁶ denotes a methyl, ethyl, or hydroxymethyl residue, or hydrogen.

6. The method according to one of Claims 1 to 5, **characterized in that** in the 1-aza-3,7-dioxabicyclo[3.3.0]octane compound of the general formula (I), the residues R² and R⁴ each denote hydrogen.

7. The method according to one of Claims 1 to 6, **characterized in that** in the 1-aza-3,7-dioxabicyclo[3.3.0]octane compound of the general formula (I), the residues R², R⁴, R⁵, R⁷ each denote hydrogen and the residue R⁶ denotes a methyl, ethyl, or hydroxymethyl residue or hydrogen; and that the residues R¹ and R³ mutually independently each denote a C₆₋₂₄ hydrocarbon residue, preferably a C₇₋₂₄ hydrocarbon residue, where the hydrocarbon residue can be acyclic or cyclic, substituted or unsubstituted, branched or unbranched, as well as saturated or unsaturated.

8. The method according to one of Claims 1 to 7, for degrading off-odors on hard and/or soft surfaces.

9. The method according to one of Claims 1 to 8 for degrading off-odors on soft surfaces, in particular textiles, in the context of a textile treatment method in which the textile is exposed, in a manual or mechanical washing or soaking process, to a washing bath that contains a 1-aza-3,7-dioxabicyclo[3.3.0]octane compound of the general formula (I), where the temperature of the washing bath is 5 to 95°C, preferably 10 to 60°C, and in particular 15 to 40°C.

10. The method according to one of Claims 1 to 9 for degrading off-odors on textiles, in which method a liquid containing a 1-aza-3,7-dioxabicyclo[3.3.0]octane compound of the general formula (I) is sprayed onto the textile.

11. The method according to one of Claims 1 to 10, in which the 1-aza-3,7-dioxabicyclo[3.3.0]octane compound of the general formula (I) is employed together with fragrances, where the 1-aza-3,7-dioxabicyclo[3.3.0]octane compound is employed in weight quantities, based on the total fragrance quantity, preferably in the range from 1:100 to 100:1, in particular from 10:1 to 1:50.

12. The method according to one of Claims 1 to 11 for degrading off-odors on hard surfaces, in which method the hard surface is brought into contact with a liquid containing a 1-aza-3,7-dioxabicyclo[3.3.0]octane compound of the general formula (I), in particular by rinsing and/or spraying.

13. The method according to one of Claims 1 to 12 for degrading off-odors in room air, in particular in living rooms, kitchens, bathrooms, toilet areas, closets, and automobiles, where the 1-aza-3,7-dioxabicyclo[3.3.0]octane compound of the general formula (I) is introduced into the room air in particular via sticks, cards, blocks, or spray.

## Revendications

1. Procédé de réduction de mauvaises odeurs à l'aide d'un 1-aza-3,7-dioxabicyclo[3.3.0]octane de la formule générale (I) 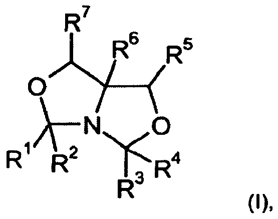 dans laquelle
R¹, R², R³, R⁴ représentent, indépendamment les uns des autres, des radicaux qui produisent, dans un composé de la formule générale R¹-C(=O)-R² ou R³-C(=O)-R⁴, un aldéhyde de parfum ayant au moins 6 atomes de carbone ou une cétone de parfum ayant au moins 6 atomes de carbone,
R⁵, R⁶, R⁷ représentent, indépendamment les uns des autres, H ou un radical hydrocarbure qui est cyclique ou acyclique, substitué ou non-substitué, ramifié ou non-ramifié et saturé ou insaturé.

2. Procédé selon la revendication 1, **caractérisé en ce que** l'aldéhyde de parfum est choisi parmi l'adoxal (2,6,10-triméthyl-9-undécénal), l'anisaldéhyde (4-méthoxybenzaldéhyde), le cymal (3-(4-isopropylphényl)-2-méthylpropanal), l'éthylvanilline, le florhydral (3-(3-isopropylphényl)butanal), l'hélional (3-(3,4-méthylènedioxyphényl)-2-méthylpropanal), l'héliotropine, l'hydroxycitronellal, le lauraldéhyde, le lyral (3- et 4-(4-hydroxy-4-méthylpentyl)-3-cyclohexén-1-carboxaldéhyde), le méthylnonyl-acétaldéhyde, le lilial (3-(4-tert-butylphényl)-2-méthylpropanal), le phénylacétaldéhyde, l'undécylénaldéhyde, la vanilline, le 2,6,10-triméthyl-9-undécénal, le 3-dodécén-1-al, l'alpha-n-amylcinnamaldéhyde, le mélonal (2,6-diméthyl-5-hepténal), le 2,4-diméthyl-3-cyclohexén-1-carboxaldéhyde (triplal), le 4-méthoxybenzaldéhyde, le benzaldéhyde, le 3-(4-tert-butylphényl)-propanal, le 2-méthyl-3-(para-méthoxyphényl)-propanal, le 2-méthyl-4-(2,6,6-triméthyl-2(1)cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzylaldéhyde, le 1,2,3,4,5,6,7,8-octahydro-8,8- diméthyl-2-naphtaldéhyde, le 2,4-diméthyl-3-cyclohexén-1-carboxaldéhyde, le 2-méthyl-3-(isopropylphényl)propanal, le décan-1-al, le 2,6-diméthyl-5-hepténal, le 4-(tricyclo[5.2.1.0 (2,6)]-décylidéne-8)-butanal, l'octahydro-4,7-méthan-1 H- indéncarboxaldéhyde, le 3-éthoxy-4-hydroxybenzaldéhyde, le para-éthyl-alpha,alpha-diméthylhydro-cinnamaldéhyde, lalpha-méthyl-3,4-(méthyléndioxy)-hydro-cinnamaldéhyde, le 3,4-méthyléndioxybenzaldéhyde, l'alpha-n-hexyl-cinnamaldéhyde, le m-cymène-7-carboxaldéhyde, l'alpha-méthylphénylacétaldéhyde, le 7-hydroxy-3,7-diméthyloctanal, l'undécénal,2,4,6-triméthyl-3-cyclohexén-1-carboxylaldéhyde, le 4-(3)(4-méthyl-3-pentényl)-3-cyclohexéncarboxaldéhyde, le 1-dodécanal, le 2,4-diméthyl-cyclohexén-3-carboxaldéhyde, le 4-(4-hydroxy-4-méthylpentyl)-3-cylohexén-1-carboxaldéhyde, le 7-méthoxy-3,7-diméthyloctan-1-al, le 2-méthylundécanal, le 2-méthyldécanal, le 1-nonanal, 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexén-1-carboxaldéhyde, le 5- ou 6-méthoxyhexahydro-4,7-méthanindan-1- ou 2-carboxaldéhyde, le 3,7-diméthyloctan-1-al, le 1-undécanal, le 10-undécén-1-al, le 4-hydroxy-3-méthoxybenzaldéhyde, le 1-méthyl-3-(4-méthylpentyl)-3-cyclohexéncarboxaldéhyde, le 7-hydroxy-3,7-diméthyl-octanal, le trans-4-décénal, le 2,6-nonadiénal, le para-tolylacétaldéhyde, le 4-méthyl-phénylacétaldéhyde, le 2-méthyl-4-(2,6,6-triméthyl-1-cyclohexén-1-yl)-2-buténal, l'ortho-méthoxycinnamaldéhyde, le 3,5,6-triméthyl-3-cyclohexéncarboxaldéhyde, le 3,7-diméthyl-2-méthylén-6-octénal, le phénoxyacétaldéhyde, le 5,9-di-méthyl-4,8-décadiénal, l'aldéhyde de pivoine (6,10-diméthyl-3-oxa-5,9-undécadién-1-al), l'hexahydro-4,7-méthanindan-1-carboxaldéhyde, le 2-méthyloctanal, l'alpha-méthyl-4-(1-méthyléthyl)benzolacétaldéhyde, le 6,6-diméthyl-2-norpinén-2-propionaldéhyde, le para-méthylphénoxy-acétaldéhyde, le 2-méthyl-3-phényl-2-propén-1-al, le 3,5,5-triméthylhexanal, l'hexahydro-8,8-diméthyl-2-naphtaldéhyde, le 3-propyl-bicyclo[2.2.1]hept-5-én-2-carbaldéhyde, le 9-décénal, le 3-méthyl-5-phényl-1-pentanal, le méthylnonylacétaldéhyde, l'hexanal et le trans-2-hexénal.

3. Procédé selon la revendication 1 ou 2, **caractérisé en ce que** la cétone de parfum est choisie la méthyl-bêta-naphtyl-cétone, le musc (1,2,3,5,6,7-hexahydro-1,1,2,3,3-pentaméthyl-4H-indén-4-one), la tonalide (6-acétyl-1,1,2,4,4,7-hexamethyltétraline), l'alpha-damascone, la bêta-damascone, la delta-damascone, l'iso-damascone, la damascénone, le dihydrojasmonate de méthyle, la menthone, la carvone, le camphre, la koavone (3,4,5,6,6-pentaméthylhept-3-én-2-one), la fenchone, l'alpha-ionone, la bêta-ionone, la gamma-méthyl-ionone, la fleuramone (2-heptylcyclopentanone), la dihydrojasmone, la cis-jasmone, l'iso-E-Super (1-(1,2,3,4,5,6,7,8-octahydro-2,3,8,8-tétraméthyl-2-naphthalényl)-éthane-1-one (et isomères)), la méthylcèdrenylcétone, l'acétophénone, la méthylacétophénone, la paraméthoxyacéto-benzophénone, la méthyl-bêta-naphtylcétone, la benzylacétone, la benzophénone, la parahydroxyphénylbutanone, la cétone de céleri (3-méthyl-5-propyl-2-cyclohexénone), la 6-isopropyldécahydro-2-naphtone, la diméthylocténone, la Frescomenthe (2-butan-2-yl-cyclohexane-1-one), la 4-(1-éthoxyvinyl)-3,3,5,5-tétraméthylcyclo-hexanone, la méthylhepténone, la 2-(2-(4-méthyl-3-cyclohexén-1-yl)propyl)cyclopentanone, la 1-(p-menthén-6(2)yl)-1-propanone, la 4-(4-hydroxy-3-méth-oxyphényl)-2-butanone, le 2-acétyl-3,3-diméthylnorbornan, la 6,7-dihydro-1,1,2,3,3-pentaméthyl-4(5H)-indanone, le 4-damascol, le dulcinyl (4-(1,3-benzodioxole-5-yl)butan-2-one), l'hexalone (1-(2,6,6-triméthyl-2-cyclohexén-1-yl)-1,6-heptadién-3-one), l'isocyclémone E (1,2,3,4,5,6,7,8-octahydro-2,3,8,8-tétraméthyl-2-acétonaphtone), la méthylnonylecétone, la méthylcyclocitrone, la méthyllavendelcétone, l'orivone (4-tert-amyl-cyclohexanone), la 4-tert-butylcyclohexanone, la delphone (2-pentyl-cyclopentanone), la muscone (CAS 541-91-3), la néobuténone (1- (5,5-diméthyl-1-cyclohexényle)pent-4-én-1-one), la plicatone (CAS 41724-19-0), la véloutone (2,2,5-triméthyl-5-pentyl-cyclopentane-1-one), la 2,4,4,7-tétraméthyl-oct-6-én-3-one ainsi que la tétrameran (6, 10-diméthylundécén-2-one).

4. Procédé selon l'une quelconque des revendications 1 à 3, **caractérisé en ce que**, dans le composé 1-aza-3,7-dioxabicyclo[3.3.0]octane de la formule générale (I), les radicaux R⁵ et R⁷ sont chacun, indépendamment l'un de l'autre, l'hydrogène ou un résidu hydrocarboné en C₁ à C₆, qui peut éventuellement être substitué, de préférence un radical hydrocarboné en C₁ à C₃, en particulier R⁵ et R⁷ représentent chacun l'hydrogène ou chacun un radical méthyle ou éthyle.

5. Procédé selon l'une des revendications 1 à 4, **caractérisé en ce que**, dans le composé 1-aza-3,7-dioxabicyclo[3.3.0]octane de la formule générale (I), le radical R⁶ est un radial méthyle, éthyle ou hydroxyméthyle ou l'hydrogène.

6. Procédé selon l'une des revendications 1 à 5, **caractérisé en ce que**, dans le composé 1-aza-3,7-dioxabicyclo[3.3.0]octane de la formule générale (I), les radicaux R² et R⁴ représentent chacun l'hydrogène.

7. Procédé selon l'une des revendications 1 à 6, **caractérisé en ce que**, dans le composé 1-aza-3,7-dioxabicyclo[3.3.0]octane de la formule générale (I), les radicaux R², R⁴, R⁵, R⁷ sont chacun l'hydrogène, et R⁶ représente un radical méthyle, éthyle ou hydroxyméthyle ou l'hydrogène, et **en ce que** les radicaux R¹ et R³ représentent, indépendamment l'un de l'autre, chacun un radical hydrocarboné en C₆ à C₂₄, de préférence un radical hydrocarboné en C₇ à C₂₄, le radical hydrocarboné pouvant être acyclique ou cyclique, substitué ou non-substitué, ramifié ou non-ramifié, saturé ou insaturé.

8. Procédé selon l'une des revendications 1 à 7 destiné à réduire les mauvaises odeurs sur des surfaces dures et/ou molles.

9. Procédé selon l'une des revendications 1 à 8 destiné à réduire les mauvaises odeurs sur des surfaces molles, en particulier des textiles, dans le cadre d'un procédé de traitement de textiles, dans lequel le textile est exposé dans une opération de lavage ou trempage à la main ou à la machine à une lessive qui contient un composé 1-aza-3,7-dioxabicyclo[3.3.0]octane de la formule générale (I), la température de la lessive allant de 5 à 95°C, de préférence de 10 à 60°C et en particulier de 15 à 40°C.

10. Procédé selon l'une des revendications 1 à 9, destiné à réduire les mauvaises odeurs sur des tissus, dans lequel un liquide, contenant un composé 1-aza-3,7-dioxabicyclo[3.3.0]octane de la formule générale (I), est pulvérisé sur le textile.

11. Procédé selon l'une des revendications 1 à 10, dans lequel le composé 1-aza-3,7-dioxabicyclo[3.3.0]octane de la formule générale (I) est utilisé conjointement avec des substances odorantes, le composé 1-aza-3,7-dioxabicyclo[3.3.0]octane de la formule générale (I) étant utilisé dans des quantités en poids, rapportées à la quantité totale de substances odorantes, de préférence dans la gamme de 1:100 à 100:1, en particulier de 10:1 à 1:50.

12. Procédé selon l'une des revendications 1 à 11 destiné à réduire les mauvaises odeurs sur des surfaces dures, dans lequel la surface dure est amenée en contact avec un liquide contenant un composé 1-aza-3,7-dioxabicyclo[3.3.0]octane de la formule générale (I), notamment par lavage et/ou par pulvérisation.

13. Procédé selon l'une des revendications 1 à 12 destiné à réduire les mauvaises odeurs dans l'air ambiant, en particulier dans les salons, les cuisines, les salles de bains, les toilettes, les placards et les véhicules automobiles, le composé 1-aza-3,7-dioxabicyclo[3.3.0]octane de la formule générale (I) étant introduit dans l'air ambiant, en particulier sous la forme des bâtonnets, de cartes, de blocs ou de spray.
